# EUROPEAN PATENT SPECIFICATION

(11) **EP 1 080 216 B1**
(45) Date of publication and mention of the grant of the patent: **01.07.2009**
(21) Application number: 99922378.7
(22) Date of filing: 21.05.1999
(51) Int. Cl.: C12N 15/86, C12N 15/62, C07K 14/16, C12N 7/01, A61K 48/00

(54) **RETROVIRAL DELIVERY SYSTEM**
RETROVIRALES VERABREICHUNGSSYSTEM
SYSTEME D'APPORT RETROVIRAL

(30) Priority: 22.05.1998 GB 9811153; 17.07.1998 US 93149 P
(43) Date of publication of application: 07.03.2001
(73) Proprietor: Oxford Biomedica (UK) Limited, Oxford 0X4 4GA (GB)
(72) Inventor: MITRAPHANOUS, Kyriacos, Andreou, Oxford OX4 4GA (GB); PATIL, Deva, University of Oxford, Oxford OX1 3QU (GB); KINGSMAN, Alan, John, Oxford BioMedica (UK) Ltd, Oxford OX4 4GA (GB); KINGSMAN, Susan Mary, Oxford BioMedica (UK) Ltd, Oxford OX4 4 GA (GB); ELLARD, Fiona Margaret, Oxford BioMedica (UK) Ltd, Oxford OX4 4GA (GB)
(74) Representative: Harding, Charles Thomas
(86) International application number: PCT/GB1999/001607
(87) International publication number: WO 1999/061639

(56) References cited:
- EP-A- 0 261 940
- WO-A-92/03537
- WO-A-92/14829
- WO-A-93/01833
- WO-A-93/14188
- WO-A-94/08022
- WO-A-95/22617
- WO-A-96/09400
- WO-A-98/05759
- P- COULON ET AL.: "An avirulent mutant of rabies virus is unable to infect motoneurons in vivo and in vitro" J. VIROLOGY, vol. 72, no. 1, January 1998 (1998-01), pages 273-278, XP002123083 AM.SOC.MICROBIOL.,WASHINGTON,US cited in the application
- REISER J ET AL: "High-titer pseudotyped HIV-1 vectors", Abstract presented at the 1997 meeting: VECTOR TARGETING STRATEGIES FOR THERAPEUTIC GENE DELIVERY, 14-16 MArch 1997

## Description

The present invention relates to a delivery system. In particular, but not exclusively, the present invention relates to an EIAV vector particle capable of delivering a nucleotide sequence of interest (hereinafter abbreviated to"NOI") - or even a plurality of NOIs - to a site of interest.

More in particular, the present invention relates to an EIAV vector particle useful in gene therapy.

The present invention also relates to a retroviral production system derivable from EIAV.

Gene therapy includes any one or more of: the addition, the replacement, the deletion, the supplementation, the manipulation etc. of one or more nucleotide sequences in, for example, one or more targetted sites-such as targetted cells. If the targetted sites are targetted cells, then the cells may be part of a tissue or an organ. General teachings on gene therapy may be found in Molecular Biology (Ed Robert Meyers, Pub VCH, such as pages 556-558).

By way of further example, gene therapy also provides a means by which any one or more of: a nucleotide sequence, such as a gene, can be applied to replace or supplement a defective gene; a pathogenic gene or gene product can be eliminated; a new gene can be added in order, for example, to create a more favourable phenotype; cells can be manipulated at the molecular level to treat cancer (Schmidt-Wolf and Schmidt-Wolf. 1994, Annals of Hematology 69; 273-279) or other conditions-such as immune, cardiovascular, neurological, inflammatory or infectious disorders; antigens can be manipulated and/or introduced to elicit an immune response-such as genetic vaccination.

In recent years, retroviruses have been proposed for use in gene therapy. Essentially, retroviruses are RNA viruses with a life cycle different to that of lytic viruses. In this regard, when a retrovirus infects a cell, its genome is converted to a DNA form. In otherwords, a retrovirus is an infectious entity that replicates through a DNA intermediate. More details on retroviral infection etc. are presented later on.

There are many retroviruses and examples include: murine leukemia virus (MLV), human immunodeficiency virus (HIV), equine infectious anaemia virus (EIAV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV).

A detailed list of retroviruses may be found in Coffin et al ("Retroviruses" 1997 Cold Spring Harbour Laboratory Press Eds: JM Coffin, SM Hughes, HE Varmus pp 758-763).

Details on the genomic structure of some retroviruses may be found in the art. By way of example, details on HIV may be found from the NCBI Genbank (i.e. Genome Accession No. AF033819).

All retroviruses contain three major coding domains, *gag, pol, env*, which code for essential virion proteins. Nevertheless, retroviruses may be broadly divided into two categories: namely, "simple" and "complex". These categories are distinguishable by the organisation of their genomes. Simple retroviruses usually carry only this elementary information. In contrast, complex retroviruses also code for additional regulatory proteins derived from multiple spliced messages.

Retroviruses may even be further divided into seven groups. Five of these groups represent retroviruses with oncogenic potential. The remaining two groups are the lentiviruses and the spumaviruses. A review of these retroviruses is presented in "Retroviruses" (1997 Cold Spring Harbour Laboratory Press Eds: JM Coffin, SM Hughes, HE Varmus pp 1-25).

All oncogenic members except the human T-cell leukemia virus-bovine leukemia virus (HTLV-BLV) are simple retroviruses. HTLV, BLV and the lentiviruses and spumaviruses are complex. Some of the best studied oncogenic retroviruses are Rous sarcoma virus (RSV), mouse mammary tumour virus (MMTV) and murine leukemia virus (MLV) and the human T-cell leukemia virus (HTLV).

The lentivirus group can be split even further into "primate" and "non-primate". Examples of primate lentiviruses include the human immunodeficiency virus (HIV), the causative agent of human auto-immunodeficiency syndrome (AIDS), and the simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiencey virus (FIV) and bovine immunodeficiencey virus (BIV).

A critical distinction between the lentivirus family and other types of retroviruses is that lentiviruses have the capability to infect both dividing and non-dividing cells (Lewis et al 1992 EMBO. J 11; 3053-3058, Lewis and Emerman 1994 J. Virol. 68: 510-516). In contrast, other retroviruses - such as MLV - are unable to infect non-dividing cells such as those that make up, for example, muscle, brain, lung and liver tissue.

During the process of infection, a retrovirus initially attaches to a specific cell surface receptor. On entry into the susceptible host cell, the retoviral RNA genome is then copied to DNA by the virally encoded reverse transcriptase which is carried inside the parent virus. This DNA is transported to the host cell nucleus where it subsequently integrates into the host genome. At this stage, it is typically referred to as the provirus. The provirus is stable in the host chromosome during cell division and is transcribed like other cellular proteins. The provirus encodes the proteins and packaging machinery required to make more virus, which can leave the cell by a process sometimes called "budding".

As already indicated, each retroviral genome comprises genes called *gag, pol* and *env* which code for virion proteins and enzymes. These genes are flanked at both ends by regions called long terminal repeats (LTRs). The LTRs are responsible for proviral integration, and transcription. They also serve as enhancer-promoter sequences. In other words, the LTRs can control the expression of the viral gene. Encapsidation of the retroviral RNAs occurs by virtue of a psi sequence located at the 5' end of the viral genome.

The LTRs themselves are indentical sequences that can be divided into three elements, which are called U3, R and U5. U3 is derived from the sequence unique to the 3' end of the RNA. R is derived from a sequence repeated at both ends of the RNA and U5 is derived from the sequence unique to the 5'end of the RNA. The sizes of the three elements can vary considerably among different retroviruses.

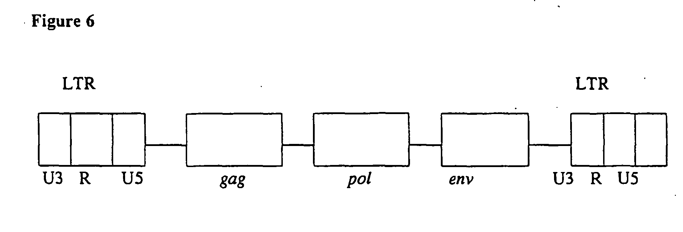

For ease of understanding, a simple, generic diagram (not to scale) of a retroviral genome showing the elementary features of the LTRs, *gag, pol* and *env* is presented in Figure 6.

For the viral genome, the site of transcription initiation is at the boundary between U3 and R in the left hand side LTR (as shown in Figure 6) and the site of poly (A) addition (termination) is at the boundary between R and U5 in the right hand side LTR (as shown in Figure 6). U3 contains most of the transcriptional control elements of the provirus, which include the promoter and multiple enhancer sequences responsive to cellular and in some cases, viral transcriptional activator proteins. Some retroviruses have any one or more of the following genes that code for proteins that are involved in the regulation of gene expression: *tat, rev, tax* and *rex*.

With regard to the structural genes *gag, pol* and *env* themselves, *gag* encodes the internal structural protein of the virus. Gag is proteolytically processed into the mature proteins MA (matrix), CA (capsid), NC (nucleocapsid). The gene *pol* encodes the reverse transcriptase (RT), which contains both DNA polymerase, and associated RNase H activities and integrase (IN), which mediates replication of the genome. The gene *env* encodes the surface (SU) glycoprotein and the transmembrane (TM) protein of the virion, which form a complex that interacts specifically with cellular receptor proteins. This interaction leads ultimately to fusion of the viral membrane with the cell membrane.

The envelope glycoprotein complex of retroviruses includes two polypeptides: an external, glycosylated hydrophilic polypeptide (SU) and a membrane-spanning protein (TM). Together, these form an oligomeric "knob" or "knobbed spike" on the surface of a virion. Both polypeptides are encoded by the *env* gene and are synthesised in the form of a polyprotein precursor that is proteolytically cleaved during its transport to the cell surface. Although uncleaved Env proteins are able to bind to the receptor, the cleavage event itself is necessary to activate the fusion potential of the protein, which is necessary for entry of the virus into the host cell. Typically, both SU and TM proteins are glycosylated at multiple sites. However, in some viruses, exemplified by MLV, TM is not glycosylated.

Although the SU and TM proteins are not always required for the assembly of enveloped virion particles as such, they do play an essential role in the entry process. In this regard, the SU domain binds to a receptor molecule - often a specific receptor molecule - on the target cell. It is believed that this binding event activates the membrane fusion-inducing potential of the TM protein after which the viral and cell membranes fuse. In some viruses, notably MLV, a cleavage event - resulting in the removal of a short portion of the cytoplasmic tail of TM - is thought to play a key role in uncovering the full fusion activity of the protein (Brody et al 1994 J. Virol. 68: 4620-4627, Rein et al 1994 J. Virol. 68: 1773-1781). This cytoplasmic "tail", distal to the membrane-spanning segment of TM remains on the internal side of the viral membrane and it varies considerably in length in different retroviruses.

Thus, the specificity of the SU/receptor interaction can define the host range and tissue tropism of a retrovirus. In some cases, this specificity may restrict the transduction potential of a recombinant retroviral vector. For this reason, many gene therapy experiments have used MLV. A particular MLV that has an envelope protein called 4070A is known as an amphotropic virus, and this can also infect human cells because its envelope protein "docks" with a phosphate transport protein that is conserved between man and mouse. This transporter is ubiquitous and so these viruses are capable of infecting many cell types. In some cases however, it may be beneficial, especially from a safety point of view, to specifically target restricted cells. To this end, several groups have engineered a mouse ecotropic retrovirus, which unlike its amphotropic relative normally only infects mouse cells, to specifically infect particular human cells. Replacement of a fragment of an envelope protein with an erythropoietin segement produced a recombinant retrovirus which then bound specifically to human cells that expressed the erythropoietin receptor on their surface, such as red blood cell precursors (Maulik and Patel 1997 "Molecular Biotechnology: Therapeutic Applications and Strategies" 1997. Wiley-Liss Inc. pp 45.).

In addition to *gag, pol* and *env*, the complex retroviruses also contain "accessory" genes which code for accessory or auxillary proteins. Accessory or auxillary proteins are defined as those proteins encoded by the accessory genes in addition to those encoded by the usual replicative or structural genes, *gag, pol* and *env.* These accessory proteins are distinct from those involved in the regulation of gene expression, like those encoded by *tat, rev, tax* and *rex*. Examples of accessory genes include one or more of *vif, vpr, vpx, vpu* and *nef*. These accessory genes can be found in, for example, HIV (see, for example pages 802 and 803 of "Retroviruses" Ed. Coffin *et al* Pub. CSHL 1997). Non-essential accessory proteins may function in specialised cell types, providing functions that are at least in part duplicative of a function provided by a cellular protein. Typically, the accessory genes are located between *pol* and *env*, just downstream from *env* including the U3 region of the LTR or overlapping portions of the *env* and each other.

The complex retroviruses have evolved regulatory mechanisms that employ virally encoded transcriptional activators as well as cellular transcriptional factors. These *trans*-acting viral proteins serve as activators of RNA transcription directed by the LTRs. The transcriptional trans-activators of the lentiviruses are encoded by the viral *tat* genes. Tat binds to a stable, stem-loop, RNA secondary structure, referred to as TAR, one function of which is to apparently optimally position Tat to *trans*-activate transcription.

As mentioned earlier, retroviruses have been proposed as a delivery system (other wise expressed as a delivery vehicle or delivery vector) for *inter alia* the transfer of a NOI, or a plurality of NOIs, to one or more sites of interest. The transfer can occur *in vitro, ex vivo, in vivo*, or combinations thereof. When used in this fashion, the retroviruses are typically called retroviral vectors or recombinant retroviral vectors. Retroviral vectors have even been exploited to study various aspects of the retrovirus life cycle, including receptor usage, reverse transcription and RNA packaging (reviewed by Miller, 1992 Curr Top Microbiol Immunol 158:1-24).

In a typical recombinant retroviral vector for use in gene therapy, at least part of one or more of the *gag, pol* and *env* protein coding regions may be removed from the virus. This makes the retroviral vector replication-defective. The removed portions may even be replaced by a NOI in order to generate a virus capable of integrating its genome into a host genome but wherein the modified viral genome is unable to propagate itself due to a lack of structural proteins. When integrated in the host genome, expression of the NOI occurs - resulting in, for example, a therapeutic effect. Thus, the transfer of a NOI into a site of interest is typically achieved by: integrating the NOI into the recombinant viral vector; packaging the modified viral vector into a virion coat; and allowing transduction of a site of interest - such as a targetted cell or a targetted cell population.

It is possible to propagate and isolate quantities of retroviral vectors (e.g. to prepare suitable titres of the retroviral vector) for subsequent transduction of, for example, a site of interest by using a combination of a packaging or helper cell line and a recombinant vector.

In some instances, propagation and isolation may entail isolation of the retroviral *gag*, *pol* and *env* genes and their separate introduction into a host cell to produce a "packaging cell line". The packaging cell line produces the proteins required for packaging retroviral DNA but it cannot bring about encapsidation due to the lack of a *psi* region. However, when a recombinant vector carrying a NOI and a psi region is introduced into the packaging cell line, the helper proteins can package the *psi*-positive recombinant vector to produce the recombinant virus stock. This can be used to infect cells to introduce the NOI into the genome of the cells. The recombinant virus whose genome lacks all genes required to make viral proteins can infect only once and cannot propagate. Hence, the NOI is introduced into the host cell genome without the generation of potentially harmful retrovirus. A summary of the available packaging lines is presented in "Retroviruses" (1997 Cold Spring Harbour Laboratory Press Eds: JM Coffin, SM Hughes, HE Varmus pp 449).

However, this technique can be problematic in the sense that the titre levels are not always at a satisfactory level. Nevertheless, the design of retroviral packaging cell lines has evolved to address the problem of *inter alia* the spontaneous production of helper virus that was frequently encountered with early designs. As recombination is greatly facilitated by homology, reducing or eliminating homology between the genomes of the vector and the helper has reduced the problem of helper virus production.

More recently, packaging cells have been developed in which the *gag, pol* and *env* viral coding regions are carried on separate expression plasmids that are independently transfected into a packaging cell line so that three recombinant events are required for wild type viral production. This strategy is sometimes referred to as the three plasmid transfection method (Soneoka et al 1995 Nucl. Acids Res. 23: 628-633).

Transient transfection can also be used to measure vector production when vectors are being developed. In this regard, transient transfection avoids the longer time required to generate stable vector-producing cell lines and is used if the vector or retroviral packaging components are toxic to cells. Components typically used to generate retroviral vectors include a plasmid encoding the Gag/Pol proteins, a plasmid encoding the Env protein and a plasmid containing a NOI. Vector production involves transient transfection of one or more of these components into cells containing the other required components. If the vector encodes toxic genes or genes that interfere with the replication of the host cell, such as inhibitors of the cell cycle or genes that induce apotosis, it may be difficult to generate stable vector-producing cell lines, but transient transfection can be used to produce the vector before the cells die. Also, cell lines have been developed using transient infection that produce vector titre levels that are comparable to the levels obtained from stable vector-producing cell lines (Pear et al 1993, PNAS 90:8392-8396).

In view of the toxicity of some HIV proteins - which can make it difficult to generate stable HIV-based packaging cells - HIV vectors are usually made by transient transfection of vector and helper virus. Some workers have even replaced the HIV Env protein with that of vesicular stomatis virus (VSV). Insertion of the Env protein of VSV facilitates vector concentration as HIV/VSV-G vectors with titres of 5 x 10⁵ (10⁸ after concentration) were generated by transient transfection (Naldini et al 1996 Science 272: 263-267). Thus, transient transfection of HIV vectors may provide a useful strategy for the generation of high titre vectors (Yee et al 1994 PNAS. 91: 9564-9568). A drawback, however, with this approach is that the VSV-G protein is quite toxic to cells.

Replacement of the *env* gene with a heterologous *env* gene is an example of a technique or strategy called pseudotyping. Pseudotyping is not a new phenomenon and examples may be found in WO-A-98/05759, WO-A-98/05754, WO-A-97/17457, WO-A-96/09400, WO-A-91/00047 and Mebatsion et al 1997 Cell 90, 841-847.

Pseudotyping can confer one or more advantages. For example, with the lentiviral vectors, the *env* gene product of the HIV based vectors would restrict these vectors to infecting only cells that express a protein called CD4. But if the *env* gene in these vectors has been substituted with *env* sequences from other RNA viruses, then they may have a broader infectious spectrum (Verma and Somia 1997 Nature 389:239-242). As just described -and by way of example - workers have pseudotyped an HIV based vector with the glycoprotein from VSV (Verma and Somia 1997 *ibid*).

Also, and by way of example, the relative fragility of the retroviral Env protein may limit the ability to concentrate retroviral vectors - and concentrating the virus may result in a poor viral recovery. To some extent this problem may be overcome by substitution of the retroviral Env protein with the more stable VSV-G protein allowing more efficient vector concentration with better yields (Naldini et al 1996. Science 272: 263-267).

However, pseudotyping with VSV-G protein may not always be desirable. This is because the VSV-G protein is cytotoxic (Chen et al 1996, Proc. Natl. Acad. Sci. 10057 and references cited therein).

Hence, it is desirable to find other proteins which are non-toxic and which can be used to pseudotype a retroviral vector.

Pseudotyping of HIV vectors with rabies G protein is disclosed in Reiser et al., although lower viral titers are obtained with rabies G protein as envelope protein than with VSV-G protein (Abstract presented at Cold Spring Harbour 1977 meeting 'Vector targeting strategies for therapeutic gene delivery', 14-16 March 1997 "High-titer pseudotyped HIV-1 vectors"). WO-A-98/05759 and WO-A-95/22617 suggest pseudotyping of MLV with rabies G protein, without providing a practical example.

The present invention seeks to provide an improved EIAV vector.

According to a first aspect of the present invention there is provided a retroviral vector production system derivable from equine infectious anaemia virus (EIAV) comprising a nucleic acid sequence which encodes at least part of a rabies glycoprotein (G) protein or a mutant, variant, derivative or fragment thereof having rabies G protein activity, a nucleic acid sequence which encodes an EIAV vector genome, and optionally one or more further nucleic acid sequences derivable from EIAV which encode the packaging components required for the generation of infective EIAV vector particles containing the genome.

The retroviral vector production system of the present invention can comprise one entity. Alternatively, the retroviral vector production system of the present invention can comprise a plurality of entities which in combination provide the retroviral vector production system of the present invention. Examples of these viral vector production systems can include but are not limited to herpesviruses and adenoviruses as described in Savard et al 1997, J Virol 71(5):4111-4117; Feng et al 1997, Nat Biotechnol 15(9): 866-870; and UK Patent Application No. 9720465.5.

The term "derivable" is used in its normal sense as meaning the sequence need not necessarily be obtained from a retrovirus but instead could be derived therefrom. By way of example, the sequence may be prepared synthetically or by use of recombinant DNA techniques.

According to a second aspect of the present invention, there is provided an EIAV vector particle obtainable from the retroviral vector production system according to the present invention.

According to a third aspect of the present invention there is provided a cell transduced with an EIAV vector particle according to the present invention.

According to a fourth aspect of the present invention there is provided an EIAV vector particle according to the present invention, for use in medicine.

According to a fifth aspect of the present invention there is provided a method comprising contacting an isolated cell with an EIAV vector particle according to the present invention.

The specification also describes a vector for preparing retroviral vector production system according to the present invention, or a viral particle according to the present invention, or a retroviral vector according to the present invention, wherein the vector comprises a nucleotide sequence coding for at least a part of the rabies G protein or a mutant, variant, derivative or fragment thereof.

The specification also describes a plasmid for preparing a retroviral vector production system according to the present invention, or a viral particle according to the present invention, or a retroviral vector according to the present invention, wherein the plasmid comprises a nucleotide sequence coding for at least a part of the rabies G protein or a mutant, variant, derivative or fragment thereof.

The specification also describes a plurality of plasmids, wherein at least one plasmid is a plasmid for use according to the present invention and wherein at lest one other plasmid comprises one or more nucleotide sequences derivable from a retrovirus.

The specification also describes the use of a rabies G protein to pseudotype a retrovirus or a retroviral vector or a retroviral particle in order to affect the infectious profile of the retrovirus or the retroviral vector or the retroviral particle.

The specification also describes the use of a rabies G protein to pseudotype a retrovirus or a retroviral vector or a retroviral particle in order to affect the host range and/or cell tropism of the retrovirus or the retroviral vector or the retroviral particle.

The specification also describes a retrovirus or a retroviral vector or a retroviral particle pseudotyped with a rabies G protein.

The specification also describes a retroviral delivery system comprising a heterologous *env* region, wherein the heterologous *env* region comprises at least a part of a nucleotide sequence coding for a rabies G protein.

The specification also describes a retroviral delivery system comprising a heterologous *env* region, wherein the heterologous *env* region comprises a nucleotide sequence coding for a rabies G protein.

Preferably the first nucleotide sequence codes for all of a rabies G protein or a mutant, variant, derivative or fragment thereof.

Preferably the retroviral delivery system comprises at least one NOI.

Preferably the NOI has a therapeutic effect or codes for a protein that has a therapeutic effect.

Preferably the target site is a cell.

An important aspect of the present invention is the pseudotyping of an EIAV vector particle derivable or based on same, with a nucleotide sequence coding for a rabies protein, especially the rabies G protein. Here, the term pseudotyping means incorporating in at least a part of, or substituting a part of, or replacing all of, an *env* gene of a viral genome, or of a viral vector, a protein from another virus.

Teachings on the rabies G protein, as well as mutants thereof, may be found in Rose et al., 1982 J. Virol. 43: 361-364, Hanham et al., 1993 J. Virol.,67, 530-542, Tuffereau et al.,1998 J. Virol., 72, 1085-1091, Kucera et al., 1985 J. Virol 55, 158-162, Dietzschold et al., 1983 PNAS 80, 70-74, Seif et al., 1985 J.Virol., 53, 926-934, Coulon et al.,1998 J. Virol., 72, 273-278, Tuffereau et al.,1998 J. Virol., 72, 1085-10910, Burger et al., 1991 J.Gen. Virol. 72. 359-367, Gaudin et al 1995 J Virol 69, 5528-5534, Benmansour et al 1991 J Virol 65, 4198-4203, Luo et al 1998 Microbiol Immunol 42, 187-193, Coll 1997 Arch Virol 142, 2089-2097, Luo et al 1997 Virus Res 51, 35-41, Luo et al 1998 Microbiol Immunol 42, 187-193, Coll 1995 Arch Virol 140, 827-851, Tuchiya et al 1992 Virus Res 25, 1-13, Morimoto et al 1992 Virology 189, 203-216, Gaudin et al 1992 Virology 187, 627-632, Whitt et al 1991 Virology 185, 681-688, Dietzschold, et al 1978 J Gen Virol 40, 131-139, Dietzschold et al 1978 Dev Biol Stand 40, 45-55, Dietzschold et al 1977 J Virol 23, 286-293, and Otvos et al 1994 Biochim Biophys Acta 1224, 68-76. A rabies G protein is also described in EP-A-0445625.

The use of rabies G protein according to the invention provides vectors which *in vivo* preferentially transduce targetted cells which rabies virus preferentially infects. This includes in particular neuronal target cells *in vivo.* For a neuron-targeted vector, rabies G from a pathogenic strain of rabies such as ERA may be particularly effective. On the other hand rabies G protein confers a wider target cell range *in vitro* including nearly all mammalian and avian cell types tested (Seganti et al., 1990 Arch Virol. 34,155-163; Fields et al., 1996 Fields Virology, Third Edition, vol.2, Lippincott-Raven Publishers, Philadelphia, New York). It is likely that it will be found also to confer an ability to infect other cell types which will be of interest. Thus, the use of rabies G protein according to the invention also enables the provision of vectors which transduce a wide variety of cell types *in vitro* and also *in vivo*. The different tropism of rabies virus *in vivo* and *in vitro* is thought to be due to the ability of rabies G to bind to a series of receptors, some of which are only active *in vitro.*

Alternatively, the tropism of the pseudotyped vector particles according to the invention may be modified by the use of a mutant rabies G which is modified in the extracellular domain. Rabies G protein has the advantage of being mutatable to restrict target cell range. The uptake of rabies virus by target cells *in vivo* is thought to be mediated by the acetylcholine receptor (AchR) but there may be other receptors to which in binds *in vivo* (Hanham et al., 1993 J. Virol.,67, 530-542; Tuffereau et al.,1998 J. Virol., 72, 1085-1091). The effects of mutations in antigenic site III of the rabies G protein on virus tropism have been investigated, this region is not thought to be involved in the binding of the virus to the acetylcholine receptor (Kucera et al., 1985 J. Virol 55, 158-162; Dietzschold et al., 1983 Proc Natl Acad Sci 80, 70-74; Seif et al., 1985 J.Virol., 53, 926-934; Coulon et al.,1998 J. Virol., 72, 273-278; Tuffereau et al.,1998 J. Virol., 72, 1085-10910). For example a mutation of the arginine at amino acid 333 in the mature protein to glutamine can be used to restrict viral entry to olfactory and peripheral neurons *in vivo* while reducing propagation to the central nervous system. These viruses were able to penetrate motorneurons and sensory neurons as efficiently as the wt virus, yet transneuronal transfer did not occur (Coulon et al., 1989, J. Virol. 63, 3550-3554). Viruses in which amino acid 330 has been mutated are further attenuated, being unable to to infect either motorneurons or sensory neurons after intramuscular injection (Coulon et al.,1998 J. Virol., 72, 273-278).

Alternatively or additionally, rabies G proteins from laboratory passaged strains of rabies may be used. These can be screened for alterations in tropism. Such strains include the following:

| **Genbank accession number** | **Rabies Strain** |
|---|---|
| J02293 | ERA |
| U52947 | COSRV |
| U27214 | NY 516 |
| U27215 | NY771 |
| U27216 | FLA125 |
| U52946 | SHBRV |
| M32751 | HEP-Flury |

By way of example, the ERA strain is a pathogenic strain of rabies and the rabies G protein from this strain can be used for transduction of neuronal cells. The sequence of rabies G from the ERA strains is in the GenBank database (accession number J02293). This protein has a signal peptide of 19 amino acids and the mature protein begins at the lysine residue 20 amino acids from the translation initiation methionine. The HEP-Flury strain contains the mutation from arginine to glutamine at amino acid position 333 in the mature protein which correlates with reduced pathogenicity and which can be used to restrict the tropism of the viral envelope.

An example of a rabies G protein is shown as SEQ ID No. 2 and its coding sequence is resented as SEQ ID No. 1. The present invention covers variants, homologues or derivatives of those sequences.

The terms "variant", "homologue" or "fragment" in relation to the amino acid sequence for the preferred enzyme of the present invention include any substitution of, variation of, modification of, replacement of, deletion of or addition of one (or more) amino acid from or to the sequence providing the resultant protein has G protein activity and/or G protein characteristics or profile, preferably being at least as biologically active as the G protein shown as SEQ ID No. 2. In particular, the term "homologue" covers homology with respect to structure and/or function. With respect to sequence homology, preferably there is at least 75%, more preferably at least 85%, more preferably at least 90% homology to the sequence shown as SEQ ID No. 2. More preferably there is at least 95%, more preferably at least 98%, homology to the sequence shown as SEQ ID No. 2.

The terms "variant", "homologue" or "fragment" in relation to the nucleotide sequence coding for the preferred enzyme of the present invention include any substitution of, variation of, modification of, replacement of, deletion of or addition of one (or more) nucleic acid from or to the sequence providing the resultant nucleotide sequence codes for or is capable of coding for a protein having G protein activity and/or G protein characteristics or profile, preferably being at least as biologically active as the G protein encoded by the sequences shown as SEQ ID No. 1. In particular, the term "homologue" covers homology with respect to structure and/or function providing the resultant nucleotide sequence codes for or is capable of coding for a protein having G protein activity and/or G protein characteristics or profile. With respect to sequence homology, preferably there is at least 75%, more preferably at least 85%, more preferably at least 90% homology to the sequence shown as SEQ ID No. 1. More preferably there is at least 95%, more preferably at least 98 %, homology to the sequence shown as SEQ ID No. 1.

In particular, the term "homology" as used herein may be equated with the term "identity". Relative sequence homology (i.e. sequence identity) can be determined by commercially available computer programs that can calculate % homology between two or more sequences. A typical example of such a computer program is CLUSTAL.

The terms "variant", "homologue" or "fragment" are synonymous with allelic variations of the sequences.

The term "variant" also encompasses sequences that are complementary to sequences that are capable of hybridising to the nucleotide sequence presented herein. Preferably, the term "variant" encompasses sequences that are complementary to sequences that are capable of hybridising under stringent conditions (e.g. 65°C and 0.1 SSC {1x SSC = 0.15 M NaCl, 0.015 Na₃ citrate pH 7.0}) to the nucleotide sequence presented herein.

The present specification also describes nucleotide sequences that can hybridise to the nucleotide sequence of the present invention (including complementary sequences of those presented herein). The present specification describes nucleotide sequences that can hybridise under stringent conditions (e.g. 65°C and 0.1 SSC) to the nucleotide sequence presented herein (including complementary sequences of those presented herein).

A major advantage of using the rabies glycoprotein in comparison to the VSV glycoprotein is the detailed knowledge of its toxicity to man and other animals due to the extensive use of rabies vaccines. In particular phase 1 clinical trials have been reported on the use of rabies glycoprotein expressed from a canarypox recombinant virus as a human vaccine (Fries et al., 1996 Vaccine 14, 428-434), these studies concluded that the vaccine was safe for use in humans.

The EIAV vector particles of the present invention are useful for the delivery of one or more NOIs to cells *in vivo* and *in vitro,* in particular the delivery of therapeutically active NOI(s). One or more selected NOI(s) may be incorporated in the vector genome for expression in the target cell. The NOI(s) may have one or more expression control sequences of their own, or their expression may be controlled by the vector LTRs. For appropriate expression of the NOI(s), a promoter may be included in or between the LTRs which is preferentially active under certain conditions or in certain cell types. The NOI may be a sense sequence or an antisense sequence. Furthermore, if there is a plurality of NOIs then those NOIs may be sense sequences or antisense sequences or combinations thereof.

The EIAV vector genome used in the present invention may generally comprise LTRs at the 5' and 3' ends, one or more NOI(s) including therapeutically active genes and/or marker genes, or suitable insertion sites for inserting one or more NOI(s), and a packaging signal to enable the genome to be packaged into a vector particle in a producer cell. There may even be suitable primer binding sites and integration sites to allow reverse transcription of the vector RNA to DNA, and integration of the proviral DNA into the target cell genome. In a preferred embodiment, the EIAV vector particle has a reverse transcription system (compatible reverse transcription and primer binding sites) and an integration system (compatible integrase and integration sites).

Thus, in accordance with the present invention, it is possible to manipulate the EIAV genome or the EIAV vector nucleotide sequence, so that viral genes are replaced or supplemented with one or more NOIs. The NOI(s) may be any one or more of selection gene(s), marker gene(s) and therapeutic gene(s). Many different selectable markers have been used successfully in retroviral vectors. These are reviewed in "Retroviruses" (1997 Cold Spring Harbour Laboratory Press Eds: JM Coffin, SM Hughes, HE Varmus pp 444) and include, but are not limited to, the bacterial neomycin and hygromycin phosphotransferase genes which confer resistance to G418 and hygromycin respectively; a mutant mouse dihydrofolate reductase gene which confers resistance to methotrexate; the bacterial gpt gene which allows cells to grow in medium containing mycophenolic acid, xanthine and aminopterin; the bacterial *hisD* gene which allows cells to grow in medium without histidine but containing histidinol; the multidrug resistance gene (*mdr*) which confers resistance to a variety of drugs; and the bacterial genes which confer resistance to puromycin or phleomycin. All of these markers are dominant selectable and allow chemical selection of most cells expressing these genes.

In accordance with the present invention, the NOI can be a therapeutic gene - in the sense that the gene itself may be capable of eliciting a therapeutic effect or it may code for a product that is capable of eliciting a therapeutic effect.

Non-limiting examples of therapeutic NOIs include genes encoding tumour supressor proteins, cytokines, anti-viral proteins, immunomodulatory molecules, antibodies, engineered immunoglobulin-like molecules, fusion proteins, hormones, membrane proteins, vasoactive proteins or peptides, growth factors, ribozymes, antisense RNA, enzymes, pro-rugs, such as pro-drug activating enzymes, cytotoxic agents, and enzyme inhibitors.

Examples of prodrugs include but are not limited to etoposide phosphate (used with alkaline phosphatase; 5-fluorocytosine (with cytosine deaminase); Doxorubin-N-p-hydroxyphenoxyacetamide (with Penicillin-V-Amidase); Para-N-bis (2-chloroethyl)aminobenzoyl glutamate (with Carboxypeptidase G2); Cephalosporin nitrogen mustard carbamates (with B-lactamase); SR4233 (with p450 reductase); Ganciclovir (with HSV thymidine kinase); mustard pro-drugs with nitroreductase and cyclophosphamide or ifosfamide (with cytochrome p450).

Diseases which may be treated include, but are not limited to cancer, heart disease, stroke, neurodegenerative disease, arthritis, viral infection, bacterial infection, parasitic infection, diseases of the immune system, viral infection, tumours, blood clotting disorders, and genetic diseases - such as chronic granulomatosis, Lesch-Nyhan sysndrome, Parkinson's disease, empysema, phenylketonuria, sickle cell anaemia, α-thalasemia, β-thalasemia, Gaucher disease.

Target cells for gene therapy using EIAV vector particles include but are not limited to haematopoietic cells, (including monocytes, macrophages, lymphocytes, granulocytes, or progenitor cells of any of these); endothelial cells, tumour cells, stromal cells, astrocytes, or glial cells, muscle cells, epithelial cells, neurons, fibroblasts, hepatocyte, astrocyte, and lung cells.

Within the EIAV vector particle of the present invention, the one or more NOIs can be under the transcriptional control of the viral LTRs. Alternatively, a combination of enhancer-promoter elements can be present in order to achieve higher levels of expression. The promoter-enhancer elements are preferably strongly active or capable of being strongly induced in the target cells. An example of a strongly active promoter-enhancer combination is a human cytomegalovirus (HCMV) major intermediate early (MIE) promoter/enhancer combination.. The promoter-enhancer combination may be tissue or temporally restricted in their activity. Examples of a suitable tissue restricted promoter-enhancer combinations are those which are highly active in tumour cells such as a promoter-enhancer combination from a MUC1 gene or a CEA gene.

Hypoxia or ischaemia regulatable expression may also be particularly useful under certain circumstances. Hypoxia is a powerful regulator of gene expression in a wide range of different cell types and acts by the induction of the activity of hypoxia-inducible transcription factors such as hypoxia inducible factor-1 (HIF-1) (Wang and Semenza 1993 PNAS. (USA) 90: 430) which bind to cognate DNA recognition sites, the hypoxia responsive elements (HREs) on various gene promoters. A multimeric form of HRE from the mouse phosphoglycerate kinase-1 (PGK-1) gene has been used to control expression of both marker and therapeutic genes by human fibrosarcoma cells in response to hypoxia *in vitro* and within solid tumours *in vivo* (Firth et al 1994. PNAS 91(14): 6496-6500; Dachs et al 1997 Nature Med. 5: 515). Alternatively, the fact that glucose deprivation is also present in ischaemic areas of tumours can be used to activate heterologous gene expression especially in tumours. A truncated 632 base pair sequence of the grp 78 gene promoter, known to be activated specifically by glucose deprivation, has been shown to be capable of driving high level expression of a reporter gene in murine rumours *in vivo* (Gazit et al 1995 Cancer Res. 55: 1660.).

The EIAV vector genomes used in the present invention for subsequent use in gene therapy preferably contain the minimum retroviral material necessary to function efficiently as vectors. The purpose of this is to allow space for the incorporation of the NOI(s), and for safety reasons. Retroviral vector genomes are preferably replication defective due to the absence of functional genes encoding one or more of the structural (or packaging) components encoded by the *gag-pol* and *env* genes. The absent components required for particle production are provided in *trans* in the producer cell. The absence of virus structural components in the genome also means that undesirable immune responses generated against virus proteins expressed in the target cell are reduced or avoided. Furthermore, possible reconstruction of infectious viral particles is preferably avoided where *in vivo* use is contemplated. Therefore, the viral structural components are preferably excluded from the genome as far as possible, in order to reduce the chance of any successful recombination.

The EIAV vector particles of the present invention are typically generated in a suitable producer cell. Producer cells are generally mammalian cells but can be for example insect cells. A producer cell may be a packaging cell containing the virus structural genes, normally integrated into its genome. The packaging cell is then transfected with a nucleic acid encoding the vector genome, for the production of infective, replication defective vector particles. Alternatively the producer cell may be co-transfected with nucleic acid sequences encoding the vector genome and the structural components, and/or with the nucleic acid sequences present on one or more expression vectors such as plasmids, adenovirus vectors, herpes viral vectors or any method known to deliver functional DNA into target cells.

We surprisingly discovered that the envelope protein from rabies virus, the rabies G protein, can efficiently pseudotype a wide variety of retroviral vectors. These include not only vectors constructed from murine oncoretroviruses such as MLV, and vectors constructed from primate lentiviruses such as HIV, but also from non-primate lentivuruses such as equine infectious anaemia virus (EIAV), which is the subject of the present invention.

The vector particle of the present invention is constructed from or is derivable from a lentivirus, namely EIAV. This has the advantage that the vector particle may be capable of transducing non-dividing cells and dividing cells.

In particular a rabies G pseudotyped lentivirus vector having rabies virus target cell range will be capable of transducing non-dividing cells of the central nervous system such as neurons.

The findings of the present invention are highly surprising. In this respect, although rabies and VSV are *Rhabdoviridae*, which is a very large family containing five diverse sub-groups, they (i.e. VSV and rabies) are in different sub-groups. Moreover, the rabies G protein has little homology with VSV-G (Rose et al., 1982 J. Virol. 43: 361-364). The rabies G protein also has a much longer cytoplasmic domain than VSV-G, of normally about 44 amino acids (in length) compared with the 28 to 31 amino acid VSV-G cytoplasmic domain. The finding that the rabies G protein is able two pseudotype MLV is therefore unexpected, given that truncation of the 144 amino acid HIV-1 cytoplasmic tail was required for efficient pseudotyping of MLV particles (Mamanano et al., 1997 J. Virol. 71:3341-3345). It is also surprising that the rabies G protein can additionally pseudotype other retroviruses such as viruses in the lentivirus group.

In other words, the invention provides the use of a rabies virus G protein to alter the target cell range of an EIAV vector particle.

In another aspect, the invention provides a method of producing EIAV vector particles having an envelope comprising rabies virus G protein, which method comprises providing a retroviral vector production system as described herein, in a producer cell, subjecting the producer cell to conditions suitable for the expression of vector particle components and the production of EIAV vector particles, and harvesting the EIAV vector particles from the supernatant.

In yet another aspect, the invention provides a method of transducing a target cell with a NOI, which method comprises contacting the cell with an EIAV vector particle as described herein, carrying the NOI, under conditions to allow attachment to and entry of the vector into the cell such that the NOI enters the target cell genome.

In addition to the rabies G protein present in the envelope of an EIAV vector particle according to the invention, one or more other envelope proteins may also be present. This may include for example a native envelope protein of the retrovirus. The use of a native envelope protein in addition to a pseudotyping protein can be beneficial to the stability and/or function of the envelope. It may even broaden the infectious profile of the vector.

We also describe a pharmaceutical composition for treating an individual by gene therapy, wherein the composition comprises a therapeutically effective amount of an EIAV vector particle according to the present invention. The pharmaceutical composition may be for human or animal usage. Typically, a physician will determine the actual dosage which will be most suitable for an individual subject and it will vary with the age, weight and response of the particular patient.

The composition may optionally comprise a pharmaceutically acceptable carrier, diluent, excipient or adjuvant. The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s), and other carrier agents that may aid or increase the viral entry into the target site (such as for example a lipid delivery system).

Where appropriate, the pharmaceutical compositions can be administered by any one or more of: inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intracavernosally, intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

Thus, in summation, the present invention relates to an EIAV vector particle having a heterologous rabies virus G protein. The present invention also relates to a retroviral vector production system for the production of EIAV vector particles having an envelope comprising a rabies virus G protein, as well as to methods of producing the vector particles and the systems, and to methods involving the use of the vector particles and the systems.

The present invention will now be described by way of example only, and with reference to the following Figures - in which:
Figure 1 presents a plasmid map;
Figure 2 presents a photograph of a gel;
Figure 3 presents a set of schematic diagrams;
Figure 4 presents a graph;
Figure 5 presents two graphs; and
Figure 6 presents a schematic diagram.

### Example 1 - Production of vectors expressing rabies G protein and details of other vectors used.

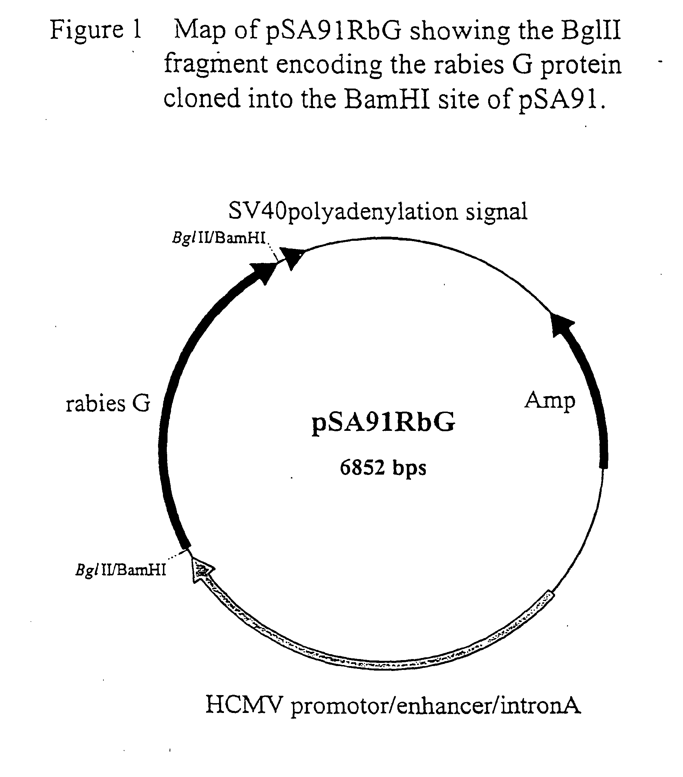

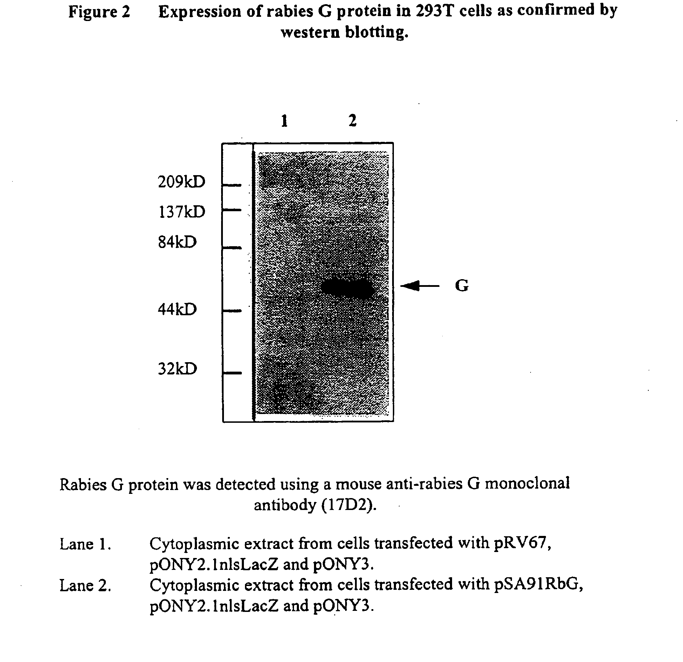

An expression vector for rabies G was constructed by cloning 1.7 kbp *Bgl*II rabies G fragment (strain ERA) from pSG5rabgp (Burger et al., 1991 J.Gen. Virol. 72. 359-367) into pSA91 (Figure 1), a derivative of pGW1HG (Soneoka et al 1995 Nucl. Acids Res. 23: 628-633) from which the *gpt* gene has been removed by digestion with *Bam*HI and re-ligation. This construct, pSA91RbG, allows expression of rabies G from the human cytomegalovirus (HCMV) immediate early gene promoter-enhancer. The plasmid was transfected, in conjunction with pONY3 (see details for construction later) and pONY2.1nlsLacZ (see details for construction later), into 293T cells by the method described by Soneoka *et al* 1995 (*ibid*) and expression of rabies G in transfected cells was confirmed by Western blotting. A VSV-G expression plasmid, in which VSV-G was expressed under the control of HCMV, in place of rabies G in pSA91 was used as a negative control. Transfected cells were washed with PBS, lysed in RIPA buffer containing 1 mM freshly prepared PMSF and total protein concentration determined using BCA protein quantification reagent kit (BCA Inc., USA) as per the manufacturer's specifications. 50 µg of cell lysate was run on 10% SDS/PAGE. The gels were electroblotted onto the immobilon membrane (Millipore Inc). Western blot analysis was carried out with 17D2, a mouse monoclonal antibody against rabies G at a dilution of 1:3000 and rabbit peroxidase conjugated anti-mouse IgG at a dilution of 1:1000 (Vector Laboratories, Peterborough, UK). Detection by chemiluminescence was performed using ECL kit (Amersham International, UK). A 68 kD band corresponding to the reported size for rabies G is apparent in cells transfected with pSA91RbG (Figure 2).

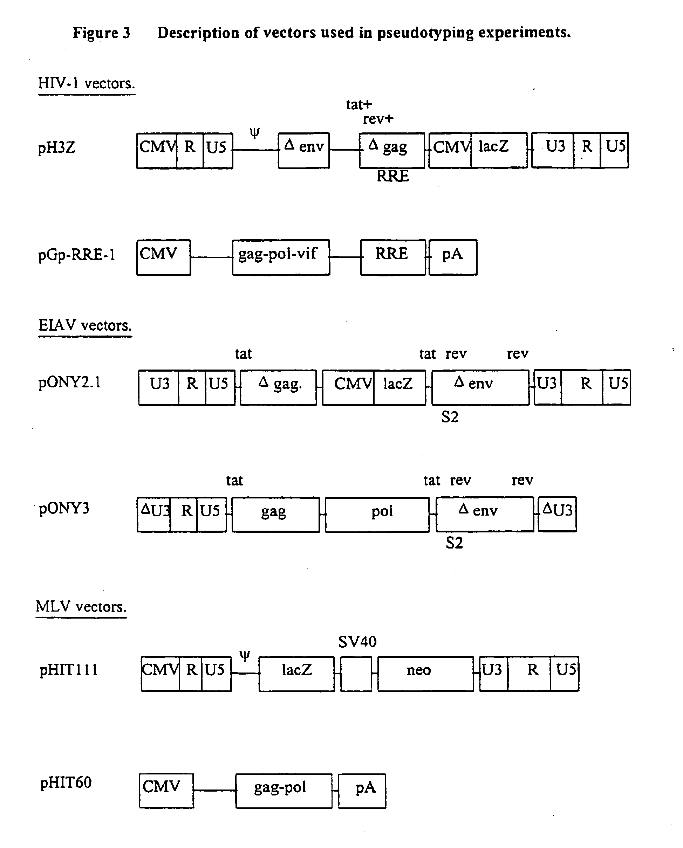

In all of the following examples the three plasmid transfection method as described previously (Soneoka *et al*., 1995, *ibid*) was used to generate pseudotyped vectors. The plasmids used in these experiments were as follows. pHIT111 and pHIT60 express an MLV vector containing the *E.coli lac*Z marker gene and MLV gag-pol respectively (Soneoka *et al*., 1995). pH3Z and pGP-RRE3 are the corresponding HIV vector components (Kim et al., 1998 J. Virol. 72:811-816). pONY2.1nlsLacZ and pONY3 are the corresponding EIAV vector components (GB patent application 9727135.7). The important features of these vectors are shown in Figure 3, which includes features of the EIAV vectors.

The description of abbreviations and labels used in Figure 3 are as follows:

| | |
|---|---|
| CMV | human cytomegalovirus immediate-early enhancer/promoter region |
| SV40 | simian virus 40 enhancer and early promoter region |
| LTR | long terminal repeat |
| R | repeat region of the LTR |
| U3 | 3 prime unique sequence of the LTR |
| ΔU3 | an incomplete U3 sequence. |
| U5 | 5 prime unique sequence of the LTR |
| ψ | genome packaging signal |
| *tat* | regulatory protein |
| *rev* | regulatory protein |
| RRE | rev response element |
| *env* | gene encoding for the envelope protein |
| Δ*env* | *env* gene containing a deletion such that a truncated protein product is produced |
| *gag* | region encoding for the capsid proteins |
| Δ*gag* | *gag* region containing a deletion such that an incomplete series of capsid proteins are produced |
| *pol* | region encoding for the enzymatic proteins |
| *vif* | viral infectivity factor |
| pA | polyadenlyation signal |
| *lacZ* | gene encoding for β-galactosidase |
| *neo* | gene encoding for neomycin phosphotransferase |

### Construction of the EIAV vectors was as follows.

An infectious proviral clone, pSPEIAV19, as described by Payne et al. (1994, J.Gen. Virol. 75:425-9) was used as a starting point. A plasmid, pSPEIAVΔH, was constructed by the deletion of a *Hind*III fragment, 5835-6571, from the region of the plasmid encoding for the envelope protein. A vector genome plasmid was constructed by inserting the EIAV LTR, amplified by PCR from pSPEIAV19, into pBluescript II KS+ (Stratagene). The *Mlu*I/*Mlu*I (216/814) fragment of pSPEIAVΔH was then inserted into the LTR/Bluescript plasmid to generate pONY2. In addition, a *Bgl*II/*Nco*I fragment within *pol* (1901/4949) was deleted and a nuclear localising β-galactosidase gene driven by the HCMV IE enhancer/promoter was inserted in its place. This was designated pONY2.1nlsLacZ.

An EIAV plasmid (pONY3) encoding the *gagpol* genes was then made by inserting the *Mlu*I/*Mlu*I fragment from pONY2ΔH into the mammalian expression plasmid pCI-neo (Promega) such that the gag-pol protein is expressed from the HCMV IE enhancer/promoter.

| **Gag-pol expression vector** | ***lacZ*-containing vector** | **Viral vector type** |
|---|---|---|
| pHIT60 | pHIT111 | MLV |
| pGP-RRE1 | pH3Z | HIV |
| pONY3 | pONY2.1nlsLacZ | EIAV |

### Example 2 - Pseudotyping retroviral vectors with rabies G protein

The three plasmid transfection method as described previously (Soneoka *et al* 1995) was used to generate pseudotyped vectors. In initial experiments, 10 µg of pSA91RbG was co-transfected with 10 µg each of a gag-pol expressing plasmid and a retroviral vector capable of expression of the *E. coli LacZ* gene per well of a 6-well tissue culture dish. Pseudotyping with VSV-G was used as a positive control for these experiments by using 10 µg of pRV67, a VSV-G expression plasmid in which VSV-G was expressed under the control of human cytomegalovirus promoter/enhancer, in place of rabies G in pSA91.

Transfections were carried out in the human kidney cell line 293T (as described in Soneoka *et al*., 1995) to produce the vector virions. Culture supernatants were harvested 36h post transfection and filtered through 0.45 mm pore-size filters (Millipore).

In contrast with the neuronal specificity of rabies *in vivo*, it is known that laboratory strains of rabies virus can interact with a wider variety of cell lines *in vitro* (Reagan and Wunner 1985 Arch. Virol. 84:277-282). Two cell lines, BHK21 cells, a baby hamster kidney cell line and D 17 cells, a dog melanoma cell line were therefore evaluated for use as target cells for retroviral vectors pseudotyped with rabies G.

Cells were seeded into 6-well tissue culture plates the day before infection at 3 x 10⁵ cells per well. Viral supernatant prepared by transfecting 293T cells with appropriate plasmids to pseudotype EIAV, HIV-1 and MLV vectors as described above was added to these cells. Polybrene (8 µg/ml) was added to each well at the time of transduction into 1 ml of the culture supernatant used for infection. 12 hours post infection, the culture supernatant was replaced by fresh medium. To measure the viral titre, cells are washed, fixed and stained 48 hours post infection.

We could observe β-galactosidase positive colonies of D 17 cells in case of transfections with pSA91RbG and pRV67 with all of the three retroviral vector constructs. There were no β-galactosidase positive colonies in transfections without either pSA91RbG or pRV67 indicating that an envelope is required for transduction to occur. In case of infections with BHK21 cells we could observe β-galactosidase positive colonies only in case of MLV pseudotypes but not with the EIAV and HIV-1 pseudotypes. This inability could be due to post-binding blocks for EIAV and HIV-1 in this cell type.
These results establish that rabies G can pseudotype EIAV, HIV and MLV vectors.

The efficiency of pseudotyping was studied by comparing the viral titres for these pseudotyped vectors. Viral titres were estimated from the number of β-galactosidase positive colonies (Table 1).

**Table 1**

| **Relative efficiencies of pseudotyping retroviral vectors with rabies G protein** | | | | | | |
|---|---|---|---|---|---|---|
| **Viral components** | | | | | **Number of transducing particles per ml as assessed on the following cells** | |
| | | | | | **D17** | **BHK-21** |
| **Retro viral vector** | **Corresponding plasmid construct** | **Gag-pol expression plasmid** | **Envelope expression plasmid** | **Resulting pseudotyped vectors** | | |
| 1 EIAV | pONY2.1nlsLacZ | pONY3 | pRV67 | EIAV-VSVG | 8.4 x 10⁴ | <10¹ |
| 2 EIAV | pONY2.1nlsLacZ | pONY3 | pSA91Rb G | EIAV-RbG | 3.2 x 10⁴ | <10¹ |
| 3 HIV | pH4Z | pGP-RRE1 | pRV67 | HIV-VSVG | 3.0 x 10⁴ | <10¹ |
| 4 HIV | pH4Z | pGP-RRE1 | pSA91Rb G | HIV-RbG | 6.4 x 10³ | <10¹ |
| 5 MLV | pHIT111 | pHIT60 | pRV67 | MLV-VSVG | 6.3 x 10⁶ | 4.8 x 10⁶ |
| 6 MLV | pHIT111 | pHIT60 | pSA91Rb G | MLV-RbG | 1.6 x 10⁶ | 7.8 x 10⁶ |
| 7 Mock | - | - | - | - | <10¹ | <10¹ |

Viral titre for MLV pseudotyped with rabies G is comparable to that observed for VSV-G pseudotypes in both the cell lines tested (4.8 x 10⁶ and 7.8 x 10⁶, in BHK21 for VSV-G and rabies G respectively; 6.3 x 10⁶ and 1.6 x 10⁶ in D17 cells for VSV-G and rabies G respectively).

Similar results were obtained in the case of EIAV and HIV-1 pseudotyped with VSV-G and rabies G in D17 cells. In the case of EIAV viral titres for the vector pseudotyped with VSV-G and rabies G were 8.4 x 10⁴ and 3.2 x 10⁴ respectively. In HIV-1, viral titres were 3.0 x 10⁴ and 6.4 x 10³ in the case of pseudotypes with VSV-G and rabies G respectively. These results indicate that rabies G is essentially as efficient as VSV-G in pseudotyping with all of the three retroviral vectors.

Our results demonstrate that rabies G protein can pseudotype retroviral vectors EIAV, HIV-1 and MLV with very high titres comparable to those obtained with VSV-G.

### Example 3 - Production and characterisation of a rabies G protein with an altered amino acid 333.

The coding sequence of the rabies glycoprotein gene in pSA91RbG was engineered, using overlap PCR, so that the resultant protein possesses a glutamate at position 333 rather than an arginine. This change has been reported to cause attenuation and altered cell tropism in rabies viruses. The primers used for mutagenesis were as follows:
forward primer
   ⁵'GATGCTCACTACAAGTCAGTCCAGACTTGGAATGAGA³TCCTCCC
reverse primer
   ⁵'GGGAGGATCTCATTCCAAGTCTGGACTGACTTGTAGT³GAGCATC

The engineered fragment was reintroduced into pSA91Rg as a *Sph*I *Bgl*II fragment, the resultant plasmid being pSA91RM. The engineered rabies G protein produced from this vector was tested for its ability to pseudotype MLV particles. The plasmid was transfected, in conjunction with pHIT60 and pHIT111, into 293T cells as described by Soneoka *et al* 1995; pSA91Rg was used as a positive control. The supernatants from these transfections were harvested as previously described, and the ability of the two envelopes to pseudotype MLV particles was assessed by their ability to transduce either BHK-21 or a murine neuroblastoma cell line, C-1300 clone NA (Table 2).

**Table 2**

| **Characterisation of the ability of a rabies G protein with an altered amino acid 333 to pseudotype MLV particles** | | | | | | |
|---|---|---|---|---|---|---|
| **Viral components** | | | | | **Number of transducing particles per ml as assessed on the following cells** | |
| | | | | | **BHK21** | **C-1300** |
| **Retro viral vector** | **Corresponding plasmid construct** | **Gag-pol expression plasmid** | **Envelope expression plasmid** | **Resulting pseudotyped vectors** | | |
| 1 MLV | pHIT111 | pHIT60 | pSA91RbG | MLV-RbG | 4.2 x 10⁶ | 6.1 x 10⁶ |
| 2 MLV | pHIT111 | pHIT60 | pSA91RM | MLV-RMG | 3.1 x 10⁵ | 7.2 x 10⁶ |
| 3 Mock | - | - | - | - | <1 | <1 |

Although the titre obtained on BHK-21 cells, a cell line that is commonly used to produce rabies vaccines, was lower with engineered rabies G protein than with the wt, comparable titres were obtained for the two envelopes on C-1300 cells. These results indicate that the engineered protein is efficiently capable of pseudotyping MLV particles.

### Example 4 - Optimisation of titre of rabies G pseudotyped retroviral vectors

The amount of pSA91RbG DNA added to the 3-plasmid transfection system was titrated against the other two components in order to determine the optimum viral titre.

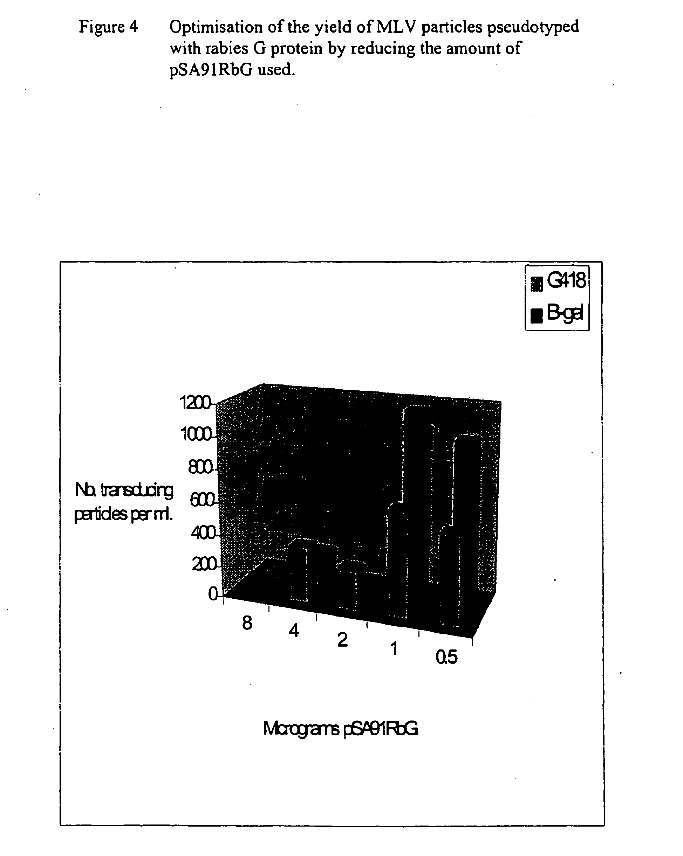

In this experiment, COS-1 cells were transfected using CaPO₄ precipitation with the plasmids, pSA91RbG, pHIT111, and pHIT60. The latter two plasmids were used in equal amounts (8 µg per transfection per 35mm tissue culture dish) whilst the amount of pSA91RbG was serially reduced. The supernatants from the transfections were harvested at 36 hours post transfection and the number of transducing particles present was determined on HT1080 cells. The results are shown in Figure 4. An eight-fold reduction in the amount of pSA91RbG DNA in comparison to the other plasmids was found to produce the highest titres. Titres overall were lower than obtained from 293T cells in Example 1.

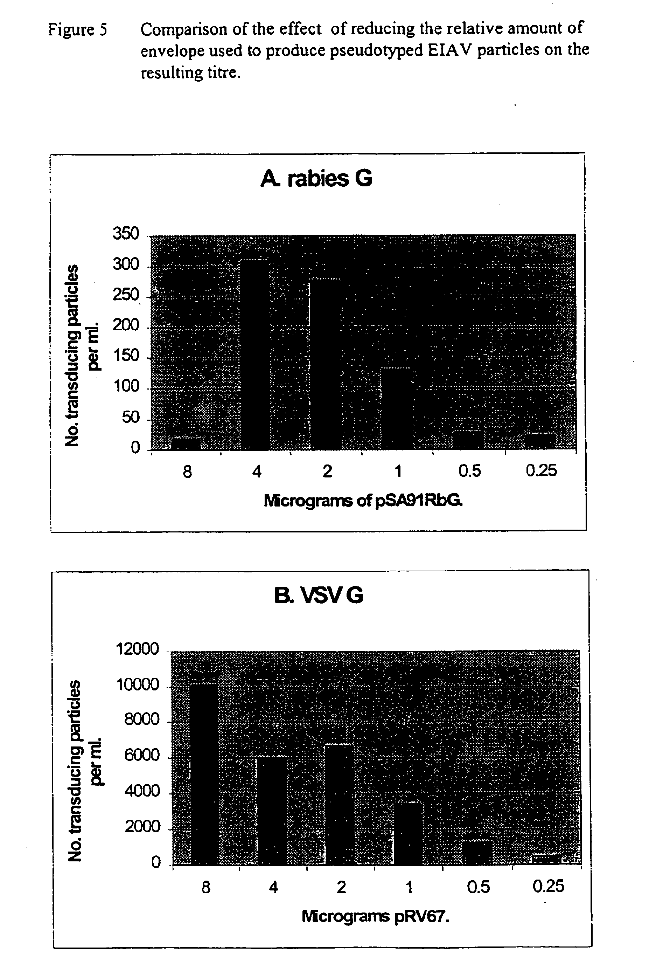

Titration of pSA91RbG against the two components required to produce EIAV-based vectors was also carried out. In this case, 293T cells were transfected using CaPO₄ precipitation with 8 µg plasmid encoding the gag/pol (pONY3) and 8 µg plasmid expressing the EIAV vector encoding for β-galactosidase (pONY2.11nlacZ). Varying amounts of pSA91RbG or pRV67 were added as above to provide vectors pseudotyped with rabies G or VSV-G respectively. The supernatants from the transfections were harvested at 36 hours post transfection and the numbers of transducing particles present was determined on D17 cells. The results are shown in Figure 5. A two- to four-fold reduction in the amount of pSA91RbG DNA in comparison to the other plasmids was found to produce the highest titres. No significant reduction in titre was observed when pRV67 was used in equal amounts to the other two plasmids.

These results show that it is possible to obtain improved titres of rabies G pseudotyped retroviral vectors by adjusting the relative amounts of rabies G and other vector components expressed in the producer cell.

### Example 5 - Concentration of rabies G pseudotyped retroviral vectors

We have further investigated whether we can increase the viral titre by concentrating the viral supernatant using ultracentrifugation (Burns et al., 1993 PNAS 90:8033-8037) obtained a viral titre of 10⁹ transducing particles per ml with VSV-G pseudotyped MLV based vector upon ultracentrifugation.

The three plasmid transfection method as described previously (Soneoka *et al*., 1995) was used to generate particles pseudotyped with either rabies G or VSV-G. Transducing particles were harvested 36 hours after transfection, concentrated by ultracentrifugation and titred on D17 cells. 48 hours later these cells were stained for β-galactosidase. Titres were averaged from three independent experiments and calculated as β-galactosidase producing units per ml. There was no more than 10% variation between experiments. It proved possible to decrease the volume of the harvests by 130 fold with out an appreciable loss of transducing particles for both EIAV and MLV particles pseudotyped with either of the two envelopes (Table 3).

**Table 3**

| **The effect of concentration on rabies G pseudotyped retroviral vectors.** | | | | | | | | | |
|---|---|---|---|---|---|---|---|---|---|
| **Viral components** | | | | | **Titre before concentration** | **Titre after concentration** | **Fold decrease in volume** | **Fold increase in titre** | **Recove ry (%)** |
| **Retro-viral vector** | **Corresponding plasmid construct** | **Gag-pol expression plasmid** | **Envelope expression plasmid** | **Resulting pseudotyped vectors** | | | | | |
| EIAV | pONY2.1nls LacZ | pONY3 | pRV67 | EIAV-VSVG | 2.0 x 10⁵ | 2.5 x 10⁷ | 130 | 125 | 96 |
| EIAV | pONY2.1nls LacZ | pONY3 | pSA91RbG | EIAV-RbG | 1.0 x 10⁵ | 1.2 x 10⁷ | 130 | 120 | 96 |
| EIAV | pONY2.1nls LacZ | pONY3 | Mock | | <1 | <1 | 130 | N.A. | N.A. |
| MLV | pHIT111 | pHIT60 | pRV67 | MLV-VSVG | 4.0x 10⁵ | 4.8 x 10⁷ | 130 | 119 | 92 |
| MLV | pHIT111 | pHIT60 | pSA91RbG | MLV-RbG | 3.5 x 10⁵ | 4.5 x 10⁷ | 130 | 128 | 98 |
| MLV | pHIT111 | pHIT60 | Mock | | <1 | <1 | 130 | N.A. | N.A. |

These results indicated that vectors pseudotyped with rabies G can be concentrated upon ultracentrifugation with an increase in the vector titre comparable to that observed for VSV-G.

These properties together with the *in vivo* specificity of the rabies G for neuronal cells make pseudotyping with the rabies G an attractive proposal for easy targeting of retroviral vectors carrying any therapeutic gene to the nervous system.

### Reference Example 6 - Selectivity of the ability of rabies G pseudotypes to transduce cultured human target cells.

The cell specificity of vectors pseudotyped with rabies G was determined using human cell lines of neuronal and non-neuronal origin. Pseudotyping with VSV-G was used as a positive control. Viral supernatant prepared from three plasmid transfection was used to infect IMR-32, a human neuroblastoma cell line and CEM-A, a human T cell line. Viral titres are estimated from β-galactosidase positive colonies (Table 4).

**Table 4**

| **Selectivity of the ability of rabies pseudotypes to transduce cultured human target cells** | | | | | | |
|---|---|---|---|---|---|---|
| **Viral components** | | | | | **Number of transducing particles per ml as assessed on the following cells** | |
| | | | | | **IMR32** | **CEM-A** |
| **Retro viral vector** | **Corresponding plasmid construct** | **Gag-pol expression plasmid** | **Envelope expression plasmid** | **Resulting pseudotyped vectors** | | |
| 1 MLV | pHIT111 | pHIT60 | pRV67 | MLV-VSVG | 4.9 x 10³ | 7.3 x 10² |
| 2 MLV | pHIT111 | pHIT60 | pSA91R bG | MLV-RbG | 3.1 x 10⁵ | 1.7 x 10¹ |
| 3 Mock | - | - | - | - | 0 | 0 |

MLV vector pseudotyped with rabies G and VSV-G could infect the IMR-32 cells. However MLV vector pseudotyped with rabies G produced titres 100 times higher (3.1 x 10⁵) than the same with VSV-G (4.9 x 10³). In CEM-A cells, MLV vector pseudotyped with rabies G gave a low titre of 1.7 x 10¹. This low efficiency was not due to the inability of MLV to transduce these cells. This is evident by the comparatively higher titre for the same vector when pseudotyped with VSV-G (7.3 x 10²).

Our results demonstrate that unlike VSV-G, rabies G pseudotypes show selectivity in their ability to transduce human target cells, with higher transduction efficiencies in neuronal cells than in the T-cell line.

### Example 7 - The ability of rabies G pseudotypes to transduce brain cells in vitro (primary cultures) and in vivo (rat/mouse model systems)

There is evidence to suggest that at least two different receptors are used by rabies virus *in vivo* (Hanham et al., 1993 J. Virol. 67:530-542; Tuffereau et al., J. Virol. 72:1085-1091), one of these receptors may be the nicotinic acetylcholine receptor. Detailed studies on the types of neuronal cells infected and the spread of the virus throughout the nervous system have been carried out with wt rabies (CVS strain) and with a double mutant of this strain (altered at amino acids 330 and 333) in mouse model systems (Coulon et al., 1989 J. Virol. 63:3550-3554; Lafay et al., 1991 Virology 183;320-330). These studies have shown that the spread and range of the mutated virus is significantly restricted in comparison to the wt.

To determine the tropism of EIAV vectors pseudotyped with rabies G protein, the following analyses are undertaken. Adult female AO rats are anaesthetized and stereotaxically injected with 2 x 1µl of viral stock into striatum or other brain regions. 7 or 30 days post-injection the rats are anaesthetized and perfused intracardially with 4% paraformaldehyde. The brains are removed, postfixed for 24 hours, saturated in 30% sucrose and sectioned on a freezing cryostat (50µm). Sections are strained with X-gal solution for 3 hours to overnight, mounted on to glass slides and analyzed with the light microscope. Identification of specific cell types transduced is made by immunofluorescence triple labeling using antibodies specific to neurons (NeuN or others), astrocytes (GFAP) or oligodendrocytes (GalC) and β-galactosidase in combination with species-specific secondary antibodies. Imaging of transduced brain regions are analyzed using confocal microscopy.

For *in vitro* transduction experiments primary neurons are put in culture from rat embryos and are grown until fully differentiated. Viral vectors are added for 5 hours using polybrene at 4µg/ml. Media are changed and expression analysis is carried out 2 days later either using X-gal staining or antibodies.

### Reference Example 8 - Concentration of rabies G pseudotyped HIV-1 vectors and comparison between the use of the VSV-G and rabies envelopes to produce such vectors

Example 5 demonstrates that ELAV vectors pseudotyped with rabies -G can be efficiently concentrated by ultracentrifugation in the same manner as has been reported with VSV-G pseudotyped vectors. We have extended these observations to show that rabies G can be used to pseudotype HIV-1 vectors, that these particles may be efficiently concentrated by ultracentrifugation and that titres obtained on D17 cells may be obtained that are equivalent to those obtained with VSV-G pseudotyped vectors.

The three plasmid transfection method as described previously (Soneoka *et al*., 1995) was used to generate particles pseudotyped with either rabies G or VSV-G. The HIV-1 plasmids, pH4Z and pGP-RRE3, used in this experiment have been discribed in Kim et al. (1998 Journal of Virology 72: 811-816). The ratio of the components used in this experiment was 1:1:1, *gag*/*pol*:genome:envelope for rabies G and 1:1:0.5, *gag*/*pol*:genome:envelope for VSV-G; this is at variance with the ratios used for COS 1 cells, since we have found that 293T cells are more resistant to the expression of the rabies G protein than COS cells. Transducing particles were harvested 48 hours after transfection, concentrated by ultracentrifugation and titred on D17 cells. 48 hours later these cells were stained for b-galactosidase. It proved possible to increase the titre of the harvests by approximately 100 with HIV-1 particles pseudotyped with either of the two envelopes (Table 5). The tires obtained for vectors pseudotyped with either of the envelopes were not significantly different. These results indicated that vectors pseudotyped with rabies G can be concentrated upon ultracentrifugation with an increase in the vector titre comparable to that observed for VSV-G and that the two envelopes are similarly effective on D17 cells.

**Table 5**

| **Preparation.** | **LacZ colony forming units per 0.5 ml.** | | | **Mean titre per ml.** |
|---|---|---|---|---|
| Rabies pseudotyped HIV-1 before concentration. | 3.4 x 10⁵ | 4.1 x 10⁵ | | 7.5 x 10⁶ |
| Rabies pseudotyped HIV-1 after concentration. | 3.5x10⁸ | 3.3x10⁸ | 3.6 x 10⁸ | 6.9 x 10⁸ |
| VSV-G pseudotyped HIV-1 before concentration. | 3.7x10⁶ | 4.0 x 10⁶ | | 7.7 x 10⁶ |
| VSV-G pseudotyped HIV-1 after concentration. | 4.8x10⁸ | 4.6x10⁸ | 3.9x10⁸ | 8.9 x 10⁸ |

### DISCUSSION AND SUMMARY

As indicated earlier, retroviruses and vector particles derived from them require a specific envelope protein in order to efficiently transduce a target cell. The envelope protein is expressed in the cell producing the virus or vector and becomes incorporated into the virus or vector particles. Retrovirus particles are composed of a proteinaceous core dervived from the *gag* gene that encases the viral RNA. The core is then encased in a portion of cell membrane that contains an envelope protein derived from the viral *env* gene. The envelope protein is produced as a precursor, which is processed into two or three units. These are the surface protein (SU) which is completely external to the envelope, the transmembrane protein (TM) which interacts with the SU and contains a membrane spanning region and a cytoplasmic tail (Coffin 1992 In The Retroviridae, Pleum Press, ed Levy). In some retroviruses a small peptide is removed from the TM. In order to act as an effective envelope protein, capable of binding to a target cell surface and mediating viral entry, the envelope protein has to interact in a precise manner with the appropriate receptor or receptors on the target cell in such a way as to result in internalisation of the viral particle in an appropriate manner to deliver the genome to the correct compartment of the cell to allow a productive infection to occur.

There have been many attempts to use the envelope derived from one virus to package a different virus, this is known as pseudotyping. The efficiency of pseudoryping is highly variable and appears to be strongly influenced by interactions between the cytoplasmic tail of the envelope and the core proteins of the viral particle. The process by which envelope proteins are recruited into budding virions is poorly understood, although it is known that the process is selective since most cellular proteins are excluded from retroviral particles (Hunter 1994 Semin. Virol. 5:71-83) and it has been recorded in some retroviruses that budding may occur in the absence of envelope proteins (Einfeld 1996 Curr. Top. Microbiol. Immunol. 214:133-176; Kräusslich and Welker 1996 Curr. Top. Microbiol. Immunol. 214:25-63). There is evidence for a precise molecular interaction between a cytoplasmic domain of the envelope protein and the viral core in some retroviruses. Januszeski et al (1997 J. Virol. 71: 3613-3619) have shown that minor deletions or substitutions in the cytoplasmic tail of the murine leukemia virus (MLV) envelope protein strongly inhibit incorporation of the envelope protein into viral particles. In the case of HIV-1, Cosson (1996 EMBO J. 15:5783-5788) has shown a direct interaction between the matrix protein of HIV-1 and the cytoplasmic domain of its envelope protein. This interaction between the matrix and envelope protein plays a key role in the incorporation of the envelope protein into budding HIV-1 virions. This is shown by the fact that visna virus can only be efficiently pseudotyped with HIV-1 Env if the amino terminus of the matrix domain of the visna virus gag polyprotein is replaced by the equivalent HIV-1 matrix domain (Dorfman et al., 1994 J. Virol. 68:1689-1696). However the situation is complex, since truncation of the HIV-1 Env is required for efficient psuedotyping of Molony murine leukemia virus (Mammano et al., 1997 J. Virol. 71:3341-3345), whilst truncation of the human foamy virus envelope protein reduced its ability to pseudotype murine leukemia virus (Lindemann et al., 1997 J. Virol. 71:4815-4820). There is also an environmental component to the interaction between the core of a retrovirus and the cytoplasmic tail of its envelope protein. Prolonged passage of EIAV in some cell lines results in a truncation of the glycoprotein, suggesting that host cell factors can select for a virus on the basis of the C-terminal domain of the envelope protein (Rice et al., 1990 J. Virol. 1990 64: 3770-3778).

These studies and those of many other workers indicate that it is not possible to predict that even closely related retroviruses may be able to pseudotype each other. Further more, if a given envelope fails to pseudotype a particular virus, it is not possible to predict the molecular changes that would confer the ability to pseudotype. Pseudotyping has met with some success, but is clearly constrained by the need for compatibility between the virus components and the heterologous envelope protein.

In the construction of retroviral vector particles it is desirable to engineer vectors with different target cell specificities to the native virus, to enable the delivery of genetic material to an expanded or altered range of cell types. One manner in which to achieve this is by engineering the virus envelope protein to alter its specificity. Another approach is to introduce a heterologous envelope protein into the vector to replace or add to the native envelope protein of the virus.

The MLV envelope protein is capable of pseudotyping a variety of different retroviruses. MLV envelope protein from an amphotropic virus allows transduction of a broad range of cell types including human cells. However, it may not always be desirable to have a retroviral vector which infects a large number of cell types.

The envelope glycoprotein (G) of Vesicular stomatis virus (VSV), a rhabdovirus, is another envelope protein which has been shown to be capable of pseudotyping certain retroviruses. The retrovirus MLV was successfully pseudotyped (Burns et al 1993 Proc. Natl. Acad. Sci. USA 90: 8033-7) and resulted in a vector having an altered host range compared to MLV in its native form. VSV-G pseudotyped vectors have been shown to infect not only mammalian cells, but also cell lines derived from fish, reptiles and insects (Burns *et al* 1993). VSV-G protein can be used to pseudotype certain retroviruses because its cytoplasmic tail is capable of interacting with the retroviral cores. VSV-G and MLV envelope proteins, both have short cytoplasmic tails, 28 to 31 and 32 amino acids respectively, and are thus of very similar length.

The provision of a non-retroviral pseudotyping envelope such as VSV-G protein gives the advantage that vector particles can be concentrated to a high titre without loss of infectivity (Akkina et al 1996 J. Virol. 70: 2581-5). Retrovirus envelope proteins are apparently unable to withstand the shearing forces during ultracentrifugation, probably because they consist of two non-covalently linked subunits. The interaction between the subunits may be disrupted by the centrifugation. In comparison the VSV glycoprotein is composed of a single unit.

VSV-G protein pseudotyping can therefore offer potential advantages. However, target cell specificities other than those achieved using VSV-G protein may also be desirable. Attempts have been made to alter the target cell range of VSV-G by engineering target sites into the protein. These attempts (Chen *et al*.) reported at the 1997 meeting on vector targeting strategies for therapeutic gene delivery (Cold Spring Harbor, USA), have not been successful. Other ways of altering retroviral vector target cell range are therefore needed.

Some attempts have been made to efficiently pseudotype retroviruses with other rhabdovirus envelopes. A single report (Reiser *et al*.), at the 1997 meeting on vector targeting strategies for therapeutic gene delivery (Cold Spring Harbor, USA), claimed that the glycoproteins of rabies virus and Mokola virus could pseudotype HIV-1 particles, but that "the titres obtained were far below the ones obtained with the VSV G protein".

The present invention seeks to overcome these problems by providing an EIAV delivery system that has been pseudotyped with a rabies protein, in particular a rabies G protein.

### SEQUENCE LISTING

### SEQ ID NO. 1

### NUCLEOTIDE SEQUENCE OF RABIES VIRUS STRAIN ERA

Genbank: locus RAVGPLS, accession M38452

1 aggaaagatg gttcctcagg ctctcctgtt tgtacccctt ctggtttttc cattgtgttt
61 tgggaaattc cctatttaca cgatactaga caagcttggt ccctggagcc cgattgacat
121 acatcacctc agctgcccaa acaatttggt agtggaggac gaaggatgca ccaacctgtc
181 agggttctcc tacatggaac ttaaagttgg atacatctta gccataaaaa tgaacgggtt
241 cacttgcaca ggcgttgtga cggaggctga aacctacact aacttcgttg gttatgtcac
301 aaccacgttc aaaagaaagc atttccgccc aacaccagat gcatgtagag ccgcgtacaa
361 ctggaagatg gccggtgacc ccagatatga agagtctcta cacaatccgt accctgacta
421 ccgctggctt cgaactgtaa aaaccaccaa ggagtctctc gttatcatat ctccaagtgt
481 agcagatttg gacccatatg acagatccct tcactcgagg gtcttcccta gcgggaagtg
541 ctcaggagta gcggtgtctt ctacctactg ctccactaac cacgattaca ccatttggat
601 gcccgagaat ccgagactag ggatgtcttg tgacattttt accaatagta gagggaagag
661 agcatccaaa gggagtgaga cttgcggctt tgtagatgaa agaggcctat ataagtcttt
721 aaaaggagca tgcaaactca agttatgtgg agttctagga cttagactta tggatggaac
781 atgggtcgcg atgcaaacat caaatgaaac caaatggtgc cctcccgatc agttggtgaa
841 cctgcacgac tttcgctcag acgaaattga gcaccttgtt gtagaggagt tggtcaggaa
901 gagagaggag tgtctggatg cactagagtc catcatgaca accaagtcag tgagtttcag
961 acgtctcagt catttaagaa aacttgtccc tgggtttgga aaagcatata ccatattcaa
1021 caagaccttg atggaagccg atgctcacta caagtcagtc agaacttgga atgagatcct
1081 cccttcaaaa gggtgtttaa gagttggggg gaggtgtcat cctcatgtga acggggtgtt
1141 tttcaatggt ataatattag gacctgacgg caatgtctta atcccagaga tgcaatcatc
1201 cctcctccag caacatatgg agttgttgga atcctcggtt atcccccttg tgcaccccct
1261 ggcagacccg tctaccgttt tcaaggacgg tgacgaggct gaggattttg ttgaagttca
1321 ccttcccgat gtgcacaatc aggtctcagg agttgacttg ggtctcccga actgggggaa
1381 gtatgtatta ctgagtgcag gggccctgac tgccttgatg ttgataattt tcctgatgac
1441 atgttgtaga agagtcaatc gatcagaacc tacgcaacac aatctcagag ggacagggag
1501 ggaggtgtca gtcactcccc aaagcgggaa gatcatatct tcatgggaat cacacaagag
1561 tgggggtgag accagactgt gaggactggc cgtcctttca acgatccaag tcctgaagat
1621 cacctcccct tgggggggttc ttttaaaaa

### SEQ ID NO. 2

### AMINO ACID SEQUENCE OF RABIES VIRUS STRAIN ERA

Genbank: locus RAVGPLS, accession M38452
PID:g333574"

MVPQALLFVPLLVFPLCFGKFPIYTILDKLGPWSPIDIHHLSCPNNLVVEDEGCTN
LSGFSYMELKVGYILAIKMNGFTCTGVVTEAETYTNFVGYVTTTFKRKHFRPTP
DACRAAYNWKMAGDPRYEESLHNPYPDYRWLRTVKTTKESLVIISPSVADLDPY
DRSLHSRVFPSGKCSGVAVSSTYCSTNHDYTIWMPENPRLGMSCDIFTNSRGKRA
SKGSETCGFVDERGLYKSLKGACKLKLCGVLGLRLMDGTWVAMQTSNETKWC
PPDQLVNLHDFRSDEIEHLVVEELVRKREECLDALESIMTTKSVSFRRLSHLRKL
VPGFGKAYTIFNKTLMEADAHYKSVRTWNEILPSKGCLRVGGRCHPHVNGVFF
NGIILGPDGNVLIPEMQSSLLQQHMELLESSVIPLVHPLADPSTVFKDGDEAEDFV
EVHLPDVHNQVSGVDLGLPNWGKYVLLSAGALTALMLIIFLMTCCRRVNRSEPT
QHNLRGTGREVSVTPQSGKIISSWESHKSGGETRL

## Claims

1. A retroviral vector production system derivable from equine infectious anaemia virus (EIAV) comprising a nucleic acid sequence which encodes at least part of a rabies glycoprotein (G) protein or a mutant, variant, derivative or fragment thereof having rabies G protein activity, a nucleic acid sequence which encodes an EIAV vector genome, and optionally one or more further nucleic acid sequences derivable from EIAV which encode the packaging components required for the generation of infective EIAV vector particles containing the genome.

2. A retroviral vector production system according to claim 1 wherein the first nucleic acid sequence codes for all of a rabies G protein or a mutant, variant, derivative or fragment thereof.

3. A retroviral vector production system according to any one of the preceding claims wherein the retroviral delivery system further comprises at least one nucleotide sequence of interest.

4. A retroviral vector production system according to claim 3 wherein the nucleotide sequence of interest has a therapeutic effect or codes for a protein that has a therapeutic effect.

5. An EIAV vector particle obtainable from the retroviral vector production system according to any one of the preceding claims.

6. A cell transduced with an EIAV vector particle according to claim 5.

7. A retroviral vector production system according to any one of claims 1 to 4, or an EIAV vector particle according to claim 5 for use in medicine.

8. A method comprising contacting an isolated cell with an EIAV vector particle according to claim 5.

9. A method of producing EIAV particles having an envelope comprising rabies G protein which method comprises providing a retroviral vector production system as claimed in any of claims 1 to 4, subjecting the retroviral vector production system to conditions suitable for the expression of vector particle components and the production of vector particles, and harvesting the vector particles from the supernatant.

10. A method of producing EIAV particles as claimed in claim 9 wherein the method comprises providing a retroviral vector production system as claimed in any of claims 1 to 4, in a producer cell, subjecting the producer cell to conditions suitable for the expression of vector particle components and the production of vector particles, and harvesting the vector particles from the supernatant.

11. A method as claimed in claim 9 or 10 wherein the supernatant is concentrated using ultracentrifugation.

## Patentansprüche

1. Retrovirales Vektorproduktionssystem, welches vom Virus der infektiösen Pferde-Anämie (EIAV) abgeleitet werden kann und eine Nukleinsäuresequenz, die wenigstens einen Teil eines Tollwutglycoprotein (G) -Proteins oder einer Mutante, einer Variante, eines Derivats oder eines Fragments davon, welche(s) Tollwut-G-Protein-Aktivität besitzt, codiert, eine Nukleinsäuresequenz, die ein EIAV-Vektorgenom codiert, und optional eine oder mehrere weitere Nukleinsäuresequenzen umfaßt, die von EIAV abgeleitet werden können und die Verpackungskomponenten codieren, die für die Erzeugung von infektiösen EIAV-Vektorpartikeln, die das Genom enthalten, erforderlich sind.

2. Retrovirales Vektorproduktionssystem nach Anspruch 1, wobei die erste Nukleinsäuresequenz ein Tollwut-G-Protein oder eine Mutante, eine Variante, ein Derivat oder ein Fragment davon vollständig codiert.

3. Retrovirales Vektorproduktionssystem nach einem der vorangegangenen Ansprüche, wobei das retrovirale Zuführungssystem weiterhin wenigstens eine interessierende Nukleotidsequenz umfaßt.

4. Retrovirales Vektorproduktionssystem nach Anspruch 3, wobei die interessierende Nukleotidsequenz eine therapeutische Wirkung hat oder ein Protein codiert, welches eine therapeutische Wirkung hat.

5. EIAV-Vektorpartikel, erhältlich aus dem retroviralen Vektorproduktionssystem nach einem der vorangegangenen Ansprüche.

6. Zelle, transduziert mit einem EIAV-Vektorpartikel nach Anspruch 5.

7. Retrovirales Vektorproduktionssystem nach einem der Ansprüche 1 bis 4 oder EIAV-Vektorpartikel nach Anspruch 5 zur Verwendung in der Medizin.

8. Verfahren, welches das Inkontaktbringen einer isolierten Zelle mit einem EIAV-Vektorpartikel nach Anspruch 5 umfaßt.

9. Verfahren zum Erzeugen von EIAV-Partikeln mit einer Hülle, die Tollwut-G-Protein umfaßt, wobei das Verfahren umfaßt, daß man ein retrovirales Vektorproduktionssystem nach einem der Ansprüche 1 bis 4 bereitstellt, das retrovirale Vektorproduktionssystem Bedingungen aussetzt, die für die Expression von Vektorpartikelkomponenten und die Produktion von Vektorpartikeln geeignet sind, und die Vektorpartikel aus dem Überstand erntet.

10. Verfahren zum Erzeugen von EIAV-Partikeln nach Anspruch 9, wobei das Verfahren umfaßt, daß man ein retrovirales Vektorproduktionssystem nach einem der Ansprüche 1 bis 4 in einer Produzentenzelle bereitstellt, die Produzentenzelle Bedingungen aussetzt, die für die Expression von Vektorpartikelkomponenten und die Produktion von Vektorpartikeln geeignet sind, und die Vektorpartikel aus dem Überstand erntet.

11. Verfahren nach Anspruch 9 oder 10, wobei der Überstand mittels Ultrazentrifugation konzentriert wird.

## Revendications

1. Système de production de vecteurs rétroviraux dérivable du virus de l'anémie infectieuse équine (VAIE) comprenant une séquence d'acide nucléique qui code au moins une partie d'une protéine de glycoprotéine (G) rabique ou d'un mutant, variant, dérivé ou fragment de celle-ci ayant une activité de protéine G rabique, une séquence d'acide nucléique qui code un génome de vecteur de VAIE, et éventuellement une ou plusieurs autres séquences d'acide nucléique dérivables de VAIE qui codent les composants d'empaquetage nécessaires pour la formation de particules de vecteur de VAIE infectieuses contenant le génome.

2. Système de production de vecteurs rétroviraux selon la revendication 1 où la première séquence d'acide nucléique code toute une protéine G rabique ou un mutant, variant, dérivé ou fragment de celle-ci.

3. Système de production de vecteurs rétroviraux selon l'une quelconque des revendications précédentes où le système de délivrance rétroviral comprend en outre au moins une séquence nucléotidique d'intérêt.

4. Système de production de vecteurs rétroviraux selon la revendication 3 où la séquence nucléotidique d'intérêt a un effet thérapeutique ou code une protéine qui a un effet thérapeutique.

5. Particule de vecteur de VAIE pouvant être obtenue à partir du système de production de vecteurs rétroviraux selon l'une quelconque des revendications précédentes.

6. Cellule transduite avec une particule de vecteur de VAIE selon la revendication 5.

7. Système de production de vecteurs rétroviraux selon l'une quelconque des revendications 1 à 4 ou particule de vecteur de VAIE selon la revendication 5 destiné à être utilisé en médecine.

8. Procédé comprenant la mise en contact d'une cellule isolée avec une particule de vecteur de VAIE selon la revendication S.

9. Procédé de production de particules de VAIE ayant une enveloppe comprenant une protéine G rabique, lequel procédé comprend la fourniture d'un système de production de vecteurs rétroviraux selon l'une quelconque des revendications 1 à 4, l'exposition du système de production de vecteurs rétroviraux à des conditions appropriées pour l'expression de composants de particules de vecteur et la production de particules de vecteur, et la récolte des particules de vecteur à partir du surnageant.

10. Procédé de production de particules de VAIE selon la revendication 9 où le procédé comprend la fourniture d'un système de production de vecteurs rétroviraux selon l'une quelconque des revendications 1 à 4, dans une cellule productrice, l'exposition de la cellule productrice à des conditions appropriées pour l'expression de composants de particules de vecteur et la production de particules de vecteur, et la récolte des particules de vecteur à partir du surnageant.

11. Procédé selon la revendication 9 ou 10 où le surnageant est concentré par ultracentrifugation.
